# EUROPEAN PATENT SPECIFICATION

(11) **EP 2 441 433 B1**
(45) Date of publication and mention of the grant of the patent: **20.04.2016**
(21) Application number: 10187448.5
(22) Date of filing: 13.10.2010
(51) Int. Cl.: A61K 8/99, A61K 35/74, C12P 23/00, C12R 1/01

(54) **Olleya marilimosa and its use in a method for the preparation of a composition comprising zeaxanthin**
Olleya marilimosa und dessen Verwendung in einem Verfahren zur Herstellung einer Zusammensetzung enthaltend Zeaxanthin
Olleya marilimosa et son utilisation dans un procédé pour la préparation d'une composition comprenant de la zéaxanthine

(43) Date of publication of application: 18.04.2012
(73) Proprietor: Vigenent Inc., Hsinchu City 300 (TW)
(72) Inventor: Dai, Ken-Shwo, 30069, Hsinchu City (TW)
(74) Representative: Epping - Hermann - Fischer

(56) References cited:
- WO-A1-99/18814
- WO-A1-03/041695
- WO-A1-2006/120400
- WO-A1-2007/067957
- WO-A1-2010/138210
- WO-A2-2004/069186
- WO-A2-2007/043046
- US-A- 3 841 967
- US-A- 3 891 504
- US-A1- 2003 108 598
- ASKER ET AL: "Mesoflavibacter zeaxanthinifaciens gen. nov., sp. nov., a novel zeaxanthin-producing marine bacterium of the family Flavobacteriaceae", SYSTEMATIC AND APPLIED MICROBIOLOGY, URBAN UND FISCHER VERLAG, DE, vol. 30, no. 4, 15 May 2007 (2007-05-15), pages 291-296, XP022079396, ISSN: 0723-2020, DOI: DOI:10.1016/J.SYAPM.2006.12.003

## Description

### BACKGROUND OF THE INVENTION

### 1. Field of the Invention

This invention provides an isolated bacteria strain Olleya marilimosa having Accession Deposit Number CCTCC M2010201 for producing zeaxanthin and a method for preparing a composition containing microbial zeaxanthin from marine bacteria Olleya marilimosa having Accession Deposit Number CCTCC M2010201. Said composition can be used for prevention and/or treatment of human skin alteration or disease comprising an extract of marine bacteria selected from *Flavobacteriaceae,* more specifically, from *Olleya marilimosa,* wherein the extract contains an effective amount of zeaxanthin. The composition can also be used in the fields of nutraceutical, food additives, cosmetics/ skin care and pharmaceuticals.

### 2. Description of the Related Art

Carotenoids are a group of lipophilic pigments found abundant in plants, algae, and bacteria. These natural pigments are responsible for the different colors (e.g., red, yellow and orange) shown in the living organisms. Over 600 carotenoids have been identified and are structurally divided into carotenes (hydrocarbon) and xanthophylls (oxygenated carotenes). Xanthophylls are yellow color pigments. With the addition of oxygen atoms, xanthophylls (e.g., lutein, neoxanthin, violaxanthin, zeaxanthin, antheraxanthin and cryptoxanthin) exhibit more hydrophilic than carotenes. Plants such as various fruits and vegetables are the natural source of xanthophylls (Sommerburg et al., (1998) Br J Ophthalmol. 82: 907-910; Deli et al., (2001) J Agric Food Chem. 49:1517-23; Molnár et al., (2005) Phytother Res. 19:700-7) because xanthophylls play important roles in plant photoprotection by decreasing the possibility of cell damages caused by excess light (Demmig et al., (1987) Plant Physiol. 84:218-224; Niyogi et al., (1998) Plant Cell. 10:1121-34; Havaux and Niyogi, (1999) Proc Natl Acad Sci USA. 96:8762-7; Morosinotto et al., (2002) J Biol Chem. 277: 36913-20). It is interesting to note that xanthophylls play essential roles in visual health (Bone et al., (1988) Invest Ophthalmol Vis Sci. 29:843-849; Handelman et al., (1988) Invest Ophthalmol Vis Sci. 29:850-855; Yeum et al., (1995) Invest Ophthalmol Vis Sci. 36:2756-2761; Hammond et al., (1997) Invest Ophthalmol Vis Sci. 38:1795-1801; Johnson et al., (2000) Am J Clin Nutr. 71:1555-1562; Bone et al., (2003) J Nutr. 133: 992-998; Ribaya-Mercado and Blumberg, (2004) J Am Coll Nutr. 23 (6 Suppl):567S-587S). In addition, xanthophylls have been reported to be associated with lower risk of cancers (Orjuela et al., (2005) Cancer Epidemiol Biomarkers Prev. 14:1433-40; Kelemen et al., (2006) Am J Clin Nutr. 83: 1401-10; Zhang et al., (2007) Nutr Cancer. 59:46-53; Tamimi et al., (2009) Cancer Res. 69: 9323-9; Lee et al., (2009) Cancer Epidemiol Biomarkers Prev. 18:1730-9) and cardiovascular diseases (Street et al., (1994) Circulation 90:1154-1161; Dwyer et al., (2001) Circulation. 103:2922-7). A broad spectrum of biological activities described above suggests that xanthophylls are bioactive substances which are health benefits to the human beings.

Zeaxanthin, a member of the xanthophylls, is one of the essential components of the macular area. It should be noted that zeaxanthin can not be synthesized in animal bodies and therefore it must be obtained from diet. For commercial purpose, zeaxanthin can be obtained via chemical synthesis (U.S. Pat. Nos. 4,952,716; 5,227,507) or via extraction from natural sources such as plants (U.S. Pat. Nos. 5,648,564; 6,784,351; 6,191,293; 7,150,890; 7,173,145) or microbes (U.S. Pat. Nos. 3,891,504; 3,951,742; 3,951,743; 5,308,759; 5427783). Due to the cost concern in the process of chemically synthesizing zeaxanthin and the health risk concern in the consumption of this artificial zeaxanthin, the natural zeaxanthin is preferential. Though it is reported that zeaxanthin can be extracted from natural sources shown as above prior art, the problems of low yield and low purity of zeaxanthin are still existed and resulted in high cost of preparing zeaxanthin. Thus, microorganism capable of producing high purity natural zeaxanthin is required.

### SUMMARY

The present invention provides an isolated bacteria strain Olleya marilimosa having Accession Deposit Number CCTCC M2010201 for producing zeaxanthin.

The present invention also provides a method for preparing a composition containing microbial zeaxanthin from marine bacteria Olleya marilimosa having Accession Deposit Number CCTCC M2010201, comprising: (a) culturing marine bacteria Olleya marilimosa having Accession Deposit Number CCTCC M2010201 in a liquid culturing medium to form pigments containing zeaxanthin; (b) separating the pigments-containing cell mass of the marine bacteria from the liquid culturing medium and collect the cell mass to obtain the composition.

### BRIEF DESCRIPTION OF THE DRAWINGS

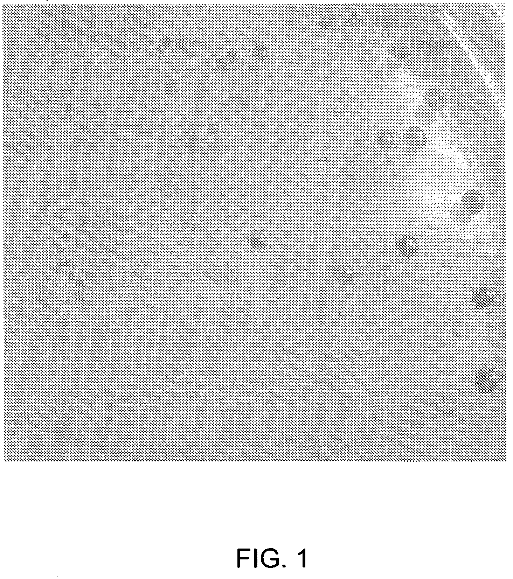

Figure 1 shows the bacterial colonies (*Olleya marilimosa* VIG2317) on agar plate.

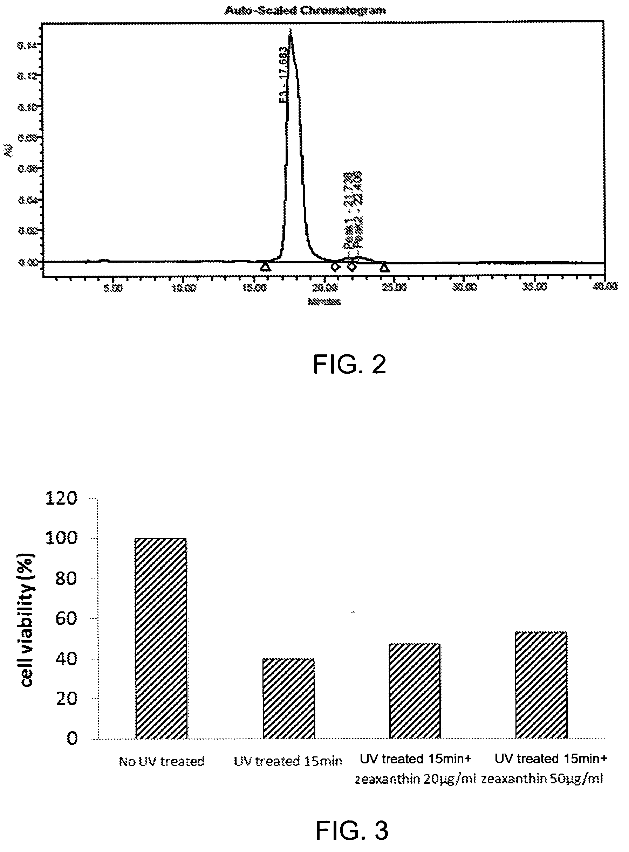

Figure 2 shows the HPLC analysis of pure microbial zeaxanthin extracted from *Olleya marilimosa* VIG2317. The purity of the microbial zeaxanthin (F3; retention time: 17.683 minutes) is greater than 90%.
Figure 3 shows the photoprotection activity of the microbial zeaxanthin extracted from *Olleya marilimosa* VIG2317. After 15 minutes of UV-irradiation, the protection effect of the microbial zeaxanthin on the viability of human skin fibroblast cells (CCD-966SK cell line) was determined at 24 hours. The cell viability treated with the microbial zeaxanthin (20µg/ml, 50µg/ml) is increased 18.9% and 33.7%, respectively, as compared to that of no zeaxanthin treatment.

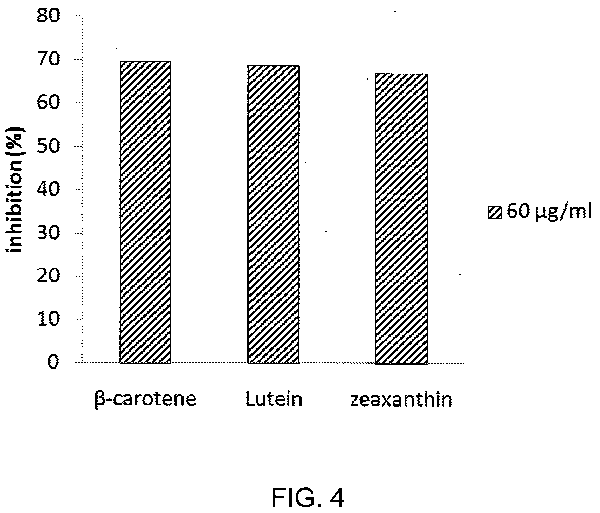

Figure 4 shows the lipid peroxidation inhibitory activity of the microbial zeaxanthin, extracted from *Olleya marilimosa* VIG2317, determined using the ferric thiocyanate method at the concentration of 60µg/ml. A comparison analysis demonstrates that the inhibitory activities (66.9%, 68.6% and 69.7%) are similar for zeaxanthin, lutein and β-carotene, respectively, in OD 500 nm.

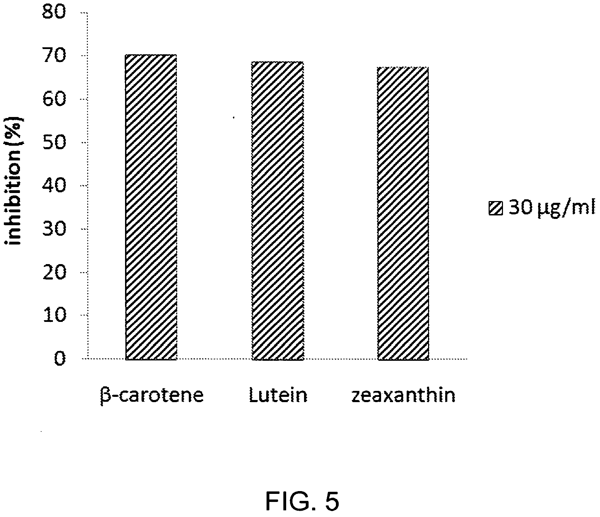

Figure 5 shows the lipid peroxidation inhibitory activity of the microbial zeaxanthin, extracted from *Olleya marilimosa* VIG2317, determined using the conjugated diene method at the concentration of 30µg/ml. A comparison analysis demonstrates that the inhibitory activities (67.4%, 68.7% and 70.3%) are similar for zeaxanthin, lutein and β-carotene, respectively, in OD 234 nm.

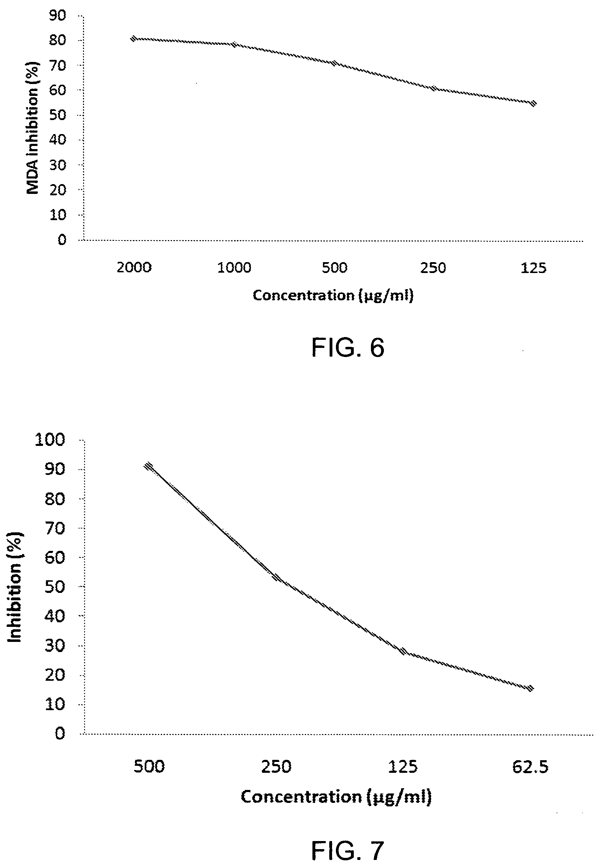

Figure 6 shows the lipid peroxidation inhibitory activity of the microbial zeaxanthin, extracted from *Olleya marilimosa* VIG2317, determined using the liposome-TBARS (Thiobarbituric acid-reactive substances) method. The percentages of inhibition on free MDA (malondialdehyde) formation determined in OD 532 nm are 55.23%, 61.05%, 71.16%, 78.45% and 80.75% for the microbial zeaxanthin at the concentrations of 125µg/ml, 250µg/ml, 500µg/ml, 1000µg/ml and 2000µg/ml, respectively.
Figure 7 shows the total antioxidant activity of the microbial zeaxanthin, extracted from *Olleya marilimosa* VIG2317, determined using the TEAC (trolox equivalent antioxidant capacity) method. The percentages of inhibition on absorbance determined in OD 734 nm are 15.68%, 28.13%, 53.35% and 91.22% for the microbial zeaxanthin at the concentrations of 62.5µg/ml, 125µg/ml, 250µg/ml, and 500µg/ml, respectively.

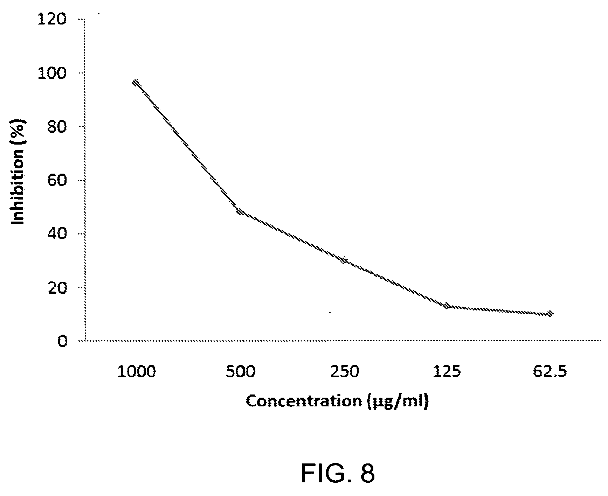

Figure 8 shows the antioxidant activity of the microbial zeaxanthin, extracted from *Olleya marilimosa* VIG2317, determined using the DPPH (1,1-diphenyl-2-picrylhydrazyl) free radical scavenging method. The percentages of inhibition on absorbance determined in OD 517 nm are 10.02%, 13.04%, 30.18%, 48.20% and 96.69% for the microbial zeaxanthin at the concentrations of 62.5µg/ml, 125µg/ml, 250µg/ml, 500µg/ml and 1000µg/ml, respectively.

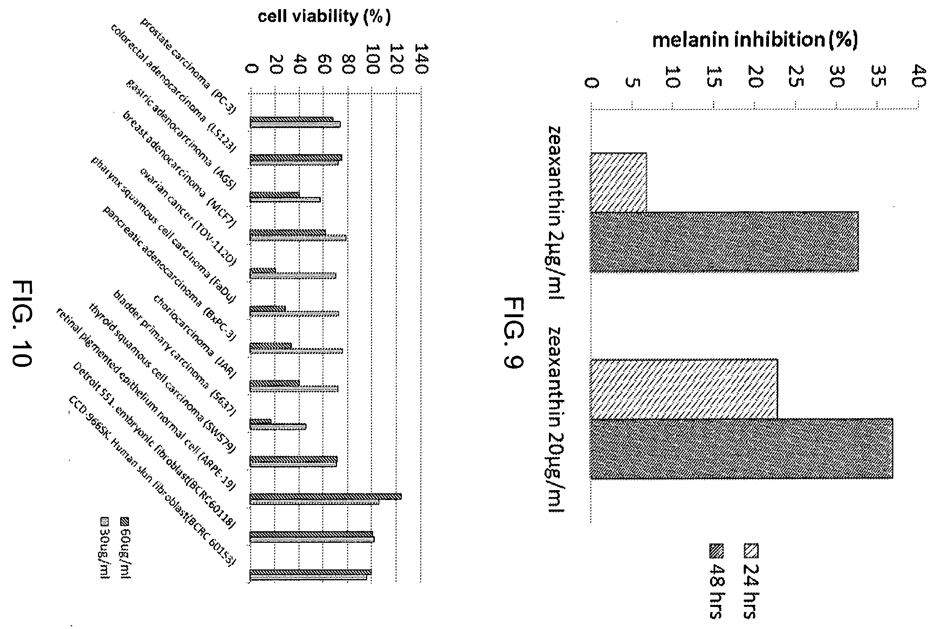

Figure 9 shows the effect of the microbial zeaxanthin, extracted from *Olleya marilimosa* VIG2317, on melanin content in B16/F10 melanoma cells. The percentages of inhibition on the melanin content determined in OD 400 nm are increased from 6.74% to 22.71% as the concentrations of the microbial zeaxanthin increased from 2 µg/ml to 20 µg/ml. When the incubation time was increased from 24 hrs to 48 hrs, percentages of inhibition on the melanin content are increased from 6.74% to 32.49% and from 22.71% to 36.74% for the concentrations of the microbial zeaxanthin at 2 µg/ml and 20 µg/ml, respectively.
Figure. 10 shows the anti-proliferation activity of the microbial zeaxanthin, extracted from *Olleya marilimosa* VIG2317, in human cancer cells using MTT assay. The cancer cell viabilities treated with 30 µg/ml and 60 µg/ml of natural zeaxanthin, respectively, are 74% and 67.7% for prostate carcinoma (PC-3); 72.3% and 75.3% for colorectal adenocarcinoma (LS123); 57.3% and 40.1% for gastric adenocarcinoma (AGS); 78.7% and 61.6% for breast adenocarcinoma (MCF7); 70.3% and 20.3% for ovarian cancer (TOV-112D); 72.6% and 28.5% for pharynx squamous cell carcinoma (FaDu); 75.6% and 33.4% for pancreatic adenocarcinoma (BxPC-3); 72.1% and 40.0% for choriocarcinoma (JAR); 45.9% and 16.4% for bladder primary carcinoma (5637) and 70.9% and 71.7% for thyroid squamous cell carcinoma (SW579). For comparison, the cell viabilities of non-cancer cells are 105.6% and 124.2% for retinal pigmented epithelium cell (ARPE-19); 102.3% and 101.2% for embryonic fibroblast (BCRC60118) and 95.8% and 99.8%for human skin fibroblast (BCRC 60153).

### DETAILED DESCRIPTION OF THE EMBODIMENTS

A composition comprising an extract of marine bacteria, containing zeaxanthin is prepared by the method of the present invention. Said composition may be used in the fields of nutraceutical, food additives, cosmetics/skin care and pharmaceuticals is provided.

The extract containing an effective amount of zeaxanthin is obtained from a strain of the marine bacteria of the family *Flavobacteriaceae.* Therefore, the zeaxanthin extracted from marine bacteria of the family *Flavobacteriaceae* is so-called "microbial zeaxanthin" in the present invention. The characteristics of marine bacteria of the family *Flavobacteriaceae* are gram-negative, yellow-pigmented, rod-shaped and aerobic. Many marine bacteria with the same characteristics are isolated from the family *Flavobacteriaceae* such as *Zeaxanthinibacter enoshimensis* (Asker et al., (2007) Int J Syst Evol Microbiol. 57(Pt 4):837-43), *Olleya marilimosa* (Nichols et al., (2005) Int J Syst Evol Microbiol. 55(Pt 4): 1557-61), *Paracoccus zeaxanthinifaciens* (Berry et al., (2003) Int J Syst Evol Microbiol. 53(Pt 1):231-8), and *Hyunsoonleella jejuensis* (Yoon et al., (2010) Int J Syst Evol Microbiol 60(Pt 2):382-6).

According to the present invention, the marine bacterium is *Olleya marilimosa.* More specifically, the marine bacterium is a novel strain, *Olleya marilimosa* VIG2317, having Accession Deposit Number CCTCC M2010201, which was deposited at the China Center for Type Culture Collection.

The composition comprising marine bacterial extract obtained from *Olleya marilimosa* VIG2317 contains from about 0.1 mg to about 10 mg as effective amount of microbial zeaxanthin, more preferably the effective amount is about 0.5 mg to about 10 mg.

As a reference, the purity of the microbial zeaxanthin obtained from *Olleya marilimosa* VIG2317 can be at least 90% (HPLC area %).

The composition of the present invention can be used to treat or prevent human skin alteration, such as skin aging resulted from exposure to UV radiation and/or skin pigmentation resulted from increased melanin content.

The composition of the present invention can be used to treat or prevent diseases such as eye diseases, cardiovascular diseases, or cancers. In one embodiment, the eye diseases are caused by insufficient levels of macular zeaxanthin and/or serum zeaxanthin. The cardiovascular diseases are associated with increased lipid peroxidation and/or malondialdehyde. The cancers are prostate carcinoma, colorectal adenocarcinoma, gastric adenocarcinoma, breast adenocarcinoma, ovarian cancer, pharynx squamous cell carcinoma, pancreatic adenocarcinoma, choriocarcinoma, bladder primary carcinoma, and/or thyroid squamous cell carcinoma.

The composition of the present invention comprises the marine bacterial extract containing microbial zeaxanthin, and a suitable carrier or excipient, wherein the carrier is, for example but not limitation, water, oil, organic solvent. The composition of the present invention can be administered topically or orally.

The present invention also provides a method for preparing a composition containing microbial zeaxanthin from marine bacteria Olleya *marilimosa* having Accession Deposit Number CCTCC M2010201, comprising the steps of: (a) culturing marine bacteria *Olleya marilimosa* having Accession Deposit Number CCTCC M2010201, comprising in a liquid culturing medium to form pigments containing zeaxanthin; (b) separating the pigments-containing cell mass of the marine bacteria with the pigments from the liquid culturing medium and collect the cell mass to obtain the composition containing microbial zeaxanthin, and (c) optionally mixing the cell mass and a carrier. The composition can be directly used as food additive or nutrient without further purification.

In one embodiment, the present invention further comprises the following steps: (d) pulverizing the cell mass of the marine bacteria; (e) digest the cell debris and dissolving the pigments by using a solvent; and (f) removing the solvent to obtain zeaxanthin. From above steps, the microbial zeaxanthin can be further isolated and purified, the zeaxanthin obtained from step (f) has at least 90% (HPLC area %) of purity. In another embodiment, the purity is at least 95%, or at least 97%.

In a reference method outside of the claimed invention, the marine bacteria is selected from a group consisting of *Zeaxanthinibacter, Olleya, Paracoccus, Hyunsoonleella,* and mutants thereof. More particulary, the marine bacteria is selected from a group consisting of *Zeaxanthinibacter enoshimensis, Olleya marilimosa, Paracoccus zeaxanthinifaciens, Hyunsoonleella jejuensis,* and mutants thereof. According to the method of the present invention, the marine bacterium is *Olleya marilimosa* VIG2317 having Accession Deposit Number CCTCC M2010201.

### EXAMPLES

The following steps are used for extracting zeaxanthin from *Olleya marilimosa* VIG2317.

### 1. Bacterial Characterization and Culture.

A bacterial strain (*Olleya marilimosa* VIG2317 having Accession Deposit Number CCTCC M2010201), isolated from a sea water sample collected from the Pacific Ocean on the east coast of Taiwan, formed yellow colonies on marine agar (FIG. 1) that had been incubated at 25°C for 2 days. The characterization of the strain VIG2317 was carried out by the Food Industry Research and Development Institute (FIRDI), Hsinchu, Taiwan. The cells are gram-negative, yellow-pigmented, rod-shaped, motile, aerobic and non-endospore-forming. According to 16S rRNA gene sequence analysis, strain VIG2317 was closely related (99% sequence similarity) to *Olleya marilimosa.* Biochemical and morphological characteristics of VIG2317 and *Olley manilimosa* CIP 108537 is shown in TABLE 1.

Individual colonies of VIG2317 on the plates were picked and cultured in rich media such as marine broth in the flask, which used as seed culture for the fermentation. A volume of seed culture was transferred to fermenter.

**TABLE 1 [0038]**

| **Characteristic** | **VIG2317** | ***Olley marilimosa* CIP 108537^{T}** |
|---|---|---|
| Growth at 25°C | + | + |
| Growth at 30°C | + | + |
| Motility | + | + |
| Production of: | | |
| Pigment | + | + |
| H₂S | - | - |
| Indole | - | - |
| β-galactosidase | - | - |
| Urease | - | - |
| Oxidase | + | + |
| Catalase | + | + |
| Acetoin | + | - |
| Arginine dihydrolase | - | - |
| β-glucosidase | + | - |
| Degraded of gelatin | + | + |
| Nitrate reduction | - | - |
| Assimilated of: | | |
| Glucose | + | + |
| Maltose | + | + |
| Mannose | + | + |
| Arabino se | - | - |
| Mannitol | - | - |
| D-gluconate | - | - |
| Capric acid | - | - |
| Adipic acid | - | - |
| Triso dium citrate | - | - |

### 2. Extraction, Purification and HPLC analysis of Microbial Zeaxanthin

Sample culture harvested from the fermenter was extracted with solvent such as acetone. The acetone extract was applied onto a silica gel column and eluted with a mixture of ethyl-acetate to hexane to 3:7(v/v). Using freeze dryer, the collected fraction was powdered for *high-performance liquid chromatograph* (HPLC) analysis.

The dried extract was dissolved in methanol and filtered for HPLC analysis. The HPLC system was programmed to inject 10 µl samples into the 4.6x250 mm ODS C-18 column. The mobile phase contained 20% methanol, 73% acetonitrile, 7% Tris-HCl buffer. The column was operated at room temperature. Separation was carried out at a flow rate of 1.0 ml/min. The detection wavelength was 450 nm. The purity of microbial zeaxanthin is greater than 90% analyzed using HPLC (FIG. 2).

### 3. Determination of Photoprotection Activity of Microbial Zeaxanthin

Human skin fibroblast cells (CCD-966SK cell line) were cultured on the dish. The cells were treated with microbial zeaxanthin dissolved in dimethyl sulfoxide. After treated for 24 hours the dishes were irradiated UV for 15 min and the cells were incubated for 24 hours. The cells were detached by trypsin and counted by hemocytometer. The protection effect of microbial zeaxanthin on the viability of fibroblast cells is provided (FIG. 3).

Skin fibroblast cells, the essential cells of skin, are responsible for the production of collagen, a structural component of the skin (Stanley et al., (1985) J Invest Dermatol. 85:542-545; Olsen et al., (1989) J. Clin. Invest. 83: 791-795; Akagi et al., (1999) J. Invest. Dermatol. 113: 246-250). Collagen reduction is a biological phenomenon responsible for the wrinkled appearance found in the aged skin caused either intrinsic (chronologic aging) or extrinsic (e.g., photoaging: exposure to UV irradiation) (Burke et al., (1994) Exp Gerontol 29:37-53; Fisher et al., (1997) New Eng J Med 337:1419-1428; Fligiel et al., (2003) J Invest Dermatol. 120:842-848; Varani et al., (2006) Am J Pathol. 168:1861-8; Chauhan and Shakya, (2009) Indian J Dermatol Venereol Leprol, 75:463-8). In addition, UV irradiation has been reported to cause loss of cell viability (Kulms and Schwarz (2000) Photodermatol Photoimmunol Photomed.16:195-201; Murphy et al., (2001) Exp. Dermatol. 10:155-160; Ichihashi et al., (2003) Toxicology. 189:21-39; Philips et al., (2007) Arch Dermatol Res. 299:373-9). After UV irradiation, the viability of human skin fibroblast cells treated with microbial zeaxanthin (20 µg/ml, 50 µg/ml) is increased 18.9% and 33.7%, respectively, as compared to that of no microbial zeaxanthin treatment. It should be noted that this increased cell viability is associated with the increased concentration of microbial zeaxanthin suggesting that microbial zeaxanthin is an important photoprotective agent which can be used in the fields of nutraceutical, food additives, cosmetics/ skin care and pharmaceuticals.

### 4. Determination of Antioxidant Activities of Microbial Zeaxanthin

The antioxidant activities of microbial zeaxanthin are provided (FIGs. 4-7). Since carotenoids are a group of organic pigments known to reduce the health risks of the biological systems via their antioxidant activities (Burton, (1989) J Nutr. 119: 109-11), it is thus essential to demonstrate if the microbial zeaxanthin is a bioactive ingredient in preventing cell damage caused by oxidation chain reactions.
(1) Lipid peroxidation inhibitory activity of the microbial zeaxanthin determined using the ferric thiocyanate method (FIG. 4):
   Microbial zeaxanthin was dissolved in 0.2 M potassium phosphate buffer and mixed with linoleic acid emulsion mixture prepared by potassium phosphate buffer. After 24 hours incubation in 37° C, the mixture was added with 75% ethanol, ammonium thiocyanate, and iron(II) chloroide tetrahydrate. The absorbance at 500 nm was determined after 1 min incubation. Inhibtion activity=[1-(Abs of sample/Abs of blank)]*100%. A comparison analysis demonstrates that the inhibitory activities (66.9%, 68.6% and 69.7%) are similar for zeaxanthin, lutein and β-carotene, respectively.
(2) Lipid peroxidation inhibitory activity of the microbial zeaxanthin determined using the conjugated diene method (FIG. 5):
   Microbial zeaxanthin was dissolved in 0.2 M potassium phosphate buffer and mixed with linoleic acid emulsion mixture prepared by potassium phosphate buffer. The absorbance of 234nm was determined after 15 hour incubation in 37°C. Inhibition activity=[1-(Abs of sample/Abs of blank)]*100%. A comparison analysis demonstrates that the inhibitory activities (67.4%, 68.7% and 70.3%) are similar for zeaxanthin, lutein and β-carotene, respectively.
(3) Lipid peroxidation inhibitory activity of the microbial zeaxanthin determined using the liposome-TBARS (Thiobarbituric acid-reactive substances) method (FIG. 6).
   Microbial zeaxanthin was dissolved in methanol and mixed with liposome emulsion, iron(III) chloride, and ascorbic acid. After 2 hours water bath in 37°C the mixture was added with butylated hydorxytoluene, thibarbituric acid, and trichloroacetic acid. After 20 min of water bath (100° C), the mixture was chilled with ice. The absorbance at 532 nm was determined. MDA (malondialdehyde) inhibiton activity= [1-(Abs of sample/Abs of blank)]* 100%. The percentages of inhibition on free MDA are increased from 55.23% to 80.75% as the concentrations of the microbial zeaxanthin increased from 125 µg/ml to 2000µg/ml.
(4) The antioxidant activity of the microbial zeaxanthin determined using the TEAC (trolox equivalent antioxidant capacity) method (FIG. 7).
   Microbial zeaxanthin was dissolved in 0.01M sodium phosphate buffer and mixed with 2,2'-azino-bis(3-ethylbenzthiazoline-6-sulphonic acid) containing potassium persulfate. The absorbance at 734 nm was determined after 30min incubation. Inhibition activity= [1-(Abs of sample/Abs of blank)]*100%. The percentages of inhibition are increased from 15.68% to 91.22% as the concentrations of the microbial zeaxanthin increased from 62.5 µg/ml to 500 µg/ml.
(5) The antioxidant activity of the microbial zeaxanthin determined using the DPPH (1,1-diphenyl-2-picrylhydrazyl) free radical scavenging method (FIG. 8).
   Microbial zeaxanthin was dissolved in methanol and mixed with 2,2-diphenyl-1-picrylhydrazyl in methanol. The absorbance at 517nm was determined after 30 min incubation. Inhibition activity= [1-(Abs of sample/Abs of blank)]*100%. The percentages of inhibition are increased from 10.02% to 96.69% as the concentrations of the microbial zeaxanthin increased from 62.5 µg/ml to 1000 µg/ml. These results suggest that the microbial zeaxanthin is a potent antioxidant, which can be used in the fields of nutraceutical, food additives, cosmetics/ skin care and pharmaceuticals.

### 5. The effect of the microbial zeaxanthin on melanin content in B16/F10 melanoma cells (FIG. 9).

B16 F0 mouse melanoma cells were cultured on the dish. The cells were treated with microbial zeaxanthin dissolved in dimethyl sulfoxide. After treated for 24 hrs and 48 hrs, respectively, the medium was removed and the cells were washed with PBS and then dissolved in 1 N NaOH containing 10% DMSO. The relative melanin content was determined at OD400nm. The percentages of inhibition on melanin content are increased from 6.74% to 22.71% as the concentrations of the microbial zeaxanthin increased from 2 µg/ml to 20 µg/ml. When the incubation time is increased from 24 hrs to 48 hrs, the inhibition are increased from 6.74% to 32.49% and from 22.71% to 36.74% for the concentrations of the microbial zeaxanthin at 2 µg/ml and 20 µg/ml, respectively. The results indicate that the microbial zeaxanthin causes dose- and time-dependent inhibiton on melanin content. This finding suggests that the microbial zeaxanthin can be used as whitening agent for cosmetics use.

### 6. The anti-proliferation activity of the microbial zeaxanthin on cancer cells (FIG. 10).

Cancer cells were cultured on the dish. The cells were treated with microbial zeaxanthin dissolved in dimethyl sulfoxide. After 24 hrs incubation, the cells were added with thiazolyl blue tetrazolium bromide and incubated for 4 hours. The medium was removed and the crystals were dissolved in dimethyl sulfoxide followed by measuring the absorbance at 590 nm. Cell viability= (Abs of cell treated zeaxanthin/Abs of cell with no treatment)* 100%. Since xanthophylls have been reported to be associated with lower risk of cancers (Orjuela et al., (2005) Cancer Epidemiol Biomarkers Prev. 14:1433-40; Kelemen et al., (2006)_Am J Clin Nutr. 83: 1401-10; Zhang et al., (2007) Nutr Cancer. 59:46-53; Tamimi et al., (2009)_Cancer Res. 69: 9323-9; Lee et al., (2009) Cancer Epidemiol Biomarkers Prev. 18:1730-9), it is necessary to determine if the microbial zeaxanthin contains anti-cancer activity. Using 30 µg/ml of the microbial zeaxanthin, the cell viabilities are 74% for prostate carcinoma (PC-3); 72.3% for colorectal adenocarcinoma (LS123); 57.3% for gastric adenocarcinoma (AGS); 78.7% for breast adenocarcinoma (MCF7); 70.3% for ovarian cancer (TOV-112D); 72.6% for pharynx squamous cell carcinoma (FaDu); 75.6% for pancreatic adenocarcinoma (BxPC-3); 72.1% for choriocarcinoma (JAR); 45.9% for bladder primary carcinoma (5637) and 70.9% for thyroid squamous cell carcinoma (SW579). For comparison, the cell viabilities of non-cancer cells are 105.6% for retinal pigmented epithelium cell (ARPE-19); 102.3% for embryonic fibroblast (BCRC60118) and 95.8% for human skin fibroblast (BCRC 60153). Under the condition of increasing the microbial zeaxanthin concentration to 60 µg/ml, the cell viabilities are 67.7% for prostate carcinoma (PC-3); 75.3% for colorectal adenocarcinoma (LS123); 40.1% for gastric adenocarcinoma (AGS); 61.6% for breast adenocarcinoma (MCF7); 20.3% for ovarian cancer (TOV-112D); 28.5% for pharynx squamous cell carcinoma (FaDu); 33.4% for pancreatic adenocarcinoma (BxPC-3); 40.0% for choriocarcinoma (JAR); 16.4% for bladder primary carcinoma (5637) and 71.7% for thyroid squamous cell carcinoma (SW579). For comparison, the cell viabilities of non-cancer cells are 124.2% for retinal pigmented epithelium cell (ARPE-19); 101.2% for embryonic fibroblast (BCRC60118) and 99.8% for human skin fibroblast (BCRC 60153). The results indicate that all cancer cells tested in the present invention are sensitive to the microbial zeaxanthin at the concentration of 30 µg/ml. However, it should be noted that dose-dependent suppression of cell proliferation is found in some cancer cells such as ovarian cancer, pharynx squamous cell carcinoma, pancreatic adenocarcinoma, choriocarcinoma, and bladder primary carcinoma when the concentration of the microbial zeaxanthin is increased to 60 µg/ml. These results suggest that the microbial zeaxanthin is an anti-cancer agent which can be used in the fields of nutraceutical, food additives, cosmetics/ skin care and pharmaceuticals.

The following formulations are provided for illustration, but not for limiting the invention.

### Formulation 1: Essence

Microbial zeaxanthin (400ppm in butylene glycol) is mixed with sodium metabisulfite, ethylhexylglycerin, phenoxyethanol, arginine, dipotassium glycyrrhizate, sodium hyaluronate, PEG-16 macadamia glycerides, acrylates/C10-30 alkyl acrylate crosspolymer, disodium EDTA and water.

### Formulation 2: Milk Lotion

Microbial zeaxanthin (400 ppm in Butylene Glycol) is mixed with sodium metabisulfite, citric acid, sodium citrate, tocopheryl acetate, cellulose gum, potassium sorbare, ethylhexylglycerin, bisabolol, ammonium acryloyldimethyltaurate/vp copolymer, butyrospermum parkii (shea butter), beheneth-25, cetyl alcohol, glycerin, cetearyl olivate, isononyl isononanoate, helianthus annuus (sunflower) seed oil, phenoxyethanol, sodium hyaluronate, disodium EDTA and water.

### Formulation 3: Dietary Supplements/Pharmaceutical Dosage

Microbial zeaxanthin 10mg is dissolved in corn oil and packaged in the form of soft gelatin capsule.

### Formulation 4: Dietary Supplements/Pharmaceutical Dosage

Microbial zeaxanthin 10mg is mixed with granulating agents and packaged in the form of tablet.

### Formulation 5: Dietary Supplements

Microbial zeaxanthin 10mg is mixed with lutein and/or other nutrients and/or antioxidants dissolved in corn oil/ peanut oil and packaged in the form of soft gelatin capsule.

### Formulation 6: Dietary Supplements

Microbial zeaxanthin 10mg is mixed with lutein and/or other nutrients and/or minerals and packaged in the form of tablet.

### REFERENCES

Akagi et al., Expression of type XVI collagen in human skin fibroblasts: enhanced expression in fibrotic skin diseases. J. Invest. Dermatol. 113: 246-250, (1999).
Asker et al., Zeaxanthinibacter enoshimensis gen. nov., sp. nov., a novel zeaxanthin-producing marine bacterium of the family Flavobacteriaceae, isolated from seawater off Enoshima Island, Japan. Int J Syst Evol Microbiol. 57(Pt 4):837-43, (2007).
Berry et al., Paracoccus zeaxanthinifaciens sp. nov., a zeaxanthin-producing bacterium. Int J Syst Evol Microbiol. 53(Pt 1):231-8, (2003).
Bone et al., Analysis of the macular pigment by HPLC: retinal distribution and age study. Invest Ophthalmol Vis Sci. 29:843 -849, (1988).
Bone et al., Lutein and zeaxanthin dietary supplements raise macular pigment density and serum concentrations of these carotenoids in humans. J Nutr1 33:992 -998, (2003).
Burke et al., Altered transcriptional regulation of human interstitial collagenase in cultured skin fibroblasts from older donors. Exp Gerontol 29:37-53, (1994).
Chauhan and Shakya, Modeling signaling pathways leading to wrinkle formation: identification of the skin aging target. Indian J Dermatol Venereol Leprol. 75:463-8, (2009).
Deli et al., Carotenoid composition in the fruits of red paprika (Capsicum annuum var. lycopersiciforme rubrum) during ripening; biosynthesis of carotenoids in red paprika. J Agric Food Chem. 49:1517-23, (2001).
Demmig et al.. Photoinhibition and Zeaxanthin Formation in Intact Leaves: A Possible Role of the Xanthophyll Cycle in the Dissipation of Excess Light Energy. Plant Physiol. 84:218-224, (1987).
Dwyer et al., Oxygenated carotenoid lutein and progression of early atherosclerosis: the Los Angeles atherosclerosis study. Circulation. 103:2922-7, (2001).
Fisher et al., Pathophysiology of premature skin aging induced by ultraviolet light. New Eng J Med 337:1419-1428, (1997).
Hammond et al., Dietary modification of human macular pigment density. Invest Ophthalmol Vis Sci 38:1795 -1801, (1997).
Handelman et al., Carotenoids in the human macula and whole retina. Invest Ophthalmol Vis Sci. 29:850 -855, (1988).
Havaux and Niyogi, The violaxanthin cycle protects plants from photooxidative damage by more than one mechanismProc Natl Acad Sci USA. 96:8762-7, (1999).
Ichihashi et al., UV-induced skin damage. Toxicology. 189:21-39, (2003).
Johnson et al., Relation among serum and tissue concentrations of lutein and zeaxanthin and macular pigment density. Am J Clin Nutr. 71:1555-1562, (2000).
Kelemen et al., Vegetables, fruit, and antioxidant-related nutrients and risk of non-Hodgkin lymphoma: a National Cancer Institute-Surveillance, Epidemiology, and End Results population-based case-control study. Am J Clin Nutr. 83: 1401-10, (2006).
Kulms and Schwarz, Molecular mechanisms of UVinduced apoptosis. Photodermatol Photoimmunol Photomed.16:195-201, (2000).
Lee et al., Intakes of fruit, vegetables, and carotenoids and renal cell cancer risk: a pooled analysis of 13 prospective studies. Cancer Epidemiol Biomarkers Prev. 18:1730-9, (2009).
Molnár et al., Biological activity of carotenoids in red paprika, Valencia orange and Golden delicious apple. Phytother Res. 19:700-7, (2005).
Morosinotto et al., Dynamics of chromophore binding to Lhc proteins in vivo and in vitro during operation of the xanthophyll cycle._J Biol Chem. 277:36913-20, (2002).
Murphy et al., The molecular determinants of sunburn cell formation. Exp Dermatol. 10:155-160, (2001).
Nichols et al., Olleya marilimosa gen. nov., sp. nov., an exopolysaccharide-producing marine bacterium from the family Flavobacteriaceae, isolated from the Southern Ocean. Int J Syst Evol Microbiol. 55(Pt 4):1557-61, (2005).
Niyogi et al., Arabidopsis mutants define a central role for the xanthophyll cycle in the regulation of photosynthetic energy conversion. Plant Cell. 10:1121-34, (1998).
Olsen et al., Collagen gene expression by cultured human skin fibroblasts. Abundant steady-state levels of type VI procollagen messenger RNAs. J. Clin. Invest. 83: 791-795, (1989).
Orjuela et al., Fruit and vegetable intake during pregnancy and risk for development of sporadic retinoblastoma. Cancer Epidemiol Biomarkers Prev. 14:1433-40, (2005).
Philips et al., Regulation of the extracellular matrix remodeling by lutein in dermal fibroblasts, melanoma cells, and ultraviolet radiation exposed fibroblasts. Arch Dermatol Res. 299:373-9, (2007).
Ribaya-Mercado and Blumberg, Lutein and zeaxanthin and their potential roles in disease prevention. J Am Coll Nutr. 23 (6 Suppl):567S-587S, (2004).
Sommerburg et al., Fruits and vegetables that are sources for lutein and zeaxanthin: the macular pigment in human eyes. Br J Ophthalmol. 82: 907-910, (1998).
Stanley et al., Epidermolysis bullosa acquisita antigen is synthesized by both human keratinocytes and human dermal fibroblasts. J Invest Dermatol. 85:542-545, (1985).
Street et al., Serum antioxidants and myocardial infarction. Are low levels of carotenoids and alpha-tocopherol risk factors for myocardial infarction? Circulation 90:1154 -1161, (1994).
Tamimi et al.,_Circulating carotenoids, mammographic density, and subsequent risk of breast cancer. Cancer Res. 69: 9323-9, (2009)._
Varani et al., Decreased collagen production in chronologically aged skin: roles of age-dependent alteration in fibroblast function and defective mechanical stimulation. Am J Pathol. 168:1861-8, (2006).
Yeum et al., Measurement of carotenoids, retinoids, and tocopherols in human lenses. Invest Ophthalmol Vis Sci. 36:2756 -2761, (1995).
Yoon et al., Hyunsoonleella jejuensis gen. nov., sp. nov., a novel member of the family Flavobacteriaceae isolated from seawater. Int J Syst Evol Microbiol. 60(Pt 2):382-6, (2010).
Zhang et al., Plasma carotenoids and prostate cancer: a population-based case-control study in Arkansas. Nutr Cancer. 59:46-53, (2007).

### PATENTS

U.S. Pat. Nos:
3,891,504: Process for the manufacture of zeaxanthin (June 24, 1975)
3,951,742: Production of zeaxanthin (April 20, 1976)
3,951,743: Production of zeaxanthin (April 20, 1976)
4,952,716: Ethynylcyclohexene compounds (August 28, 1990)
5,227,507: Ethynylcyclohexene compounds and processes for manufacture thereof (July 13, 1993)
5,308,759: Production of zeaxanthin and zeaxanthin-containing compositions (May 3, 1994)
5,427,783: Zeaxanthin-containing compositions produced by flavobacterium multivorum (June 27, 1995)
5,648,564: Process for the formation, isolation and purification of comestible xanthophyll crystals from plants (July 15, 1997)
6,784,351: Targetes erecta marigolds with altered carotenoid compositions and ratios
(August 31, 2004)
6,191,293: Trans-xanthophyll ester concentrates of enhanced purity and methods of making same (February 20, 2001)
7,150,890: Process for the purification of marigold xanthophylls (December 19, 2006)
7,173,145: Process for extraction and purification of lutein, zeaxanthin and rare carotenoids from marigold flowers and plants (February 6, 2007)

## Claims

1. An isolated bacteria strain *Olleya marilimosa* having Accession Deposit Number CCTCC M2010201 for producing zeaxanthin.

2. A method for preparing a composition comprising microbial zeaxanthin from marine bacteria *Olleya marilimosa* having Accession Deposit Number CCTCC M2010201, comprising:
(a) culturing marine bacteria *Olleya marilimosa* having Accession Deposit Number CCTCC M2010201 in a liquid culturing medium to form pigments comprising zeaxanthin;
(b) separating the pigments-containing cell mass of the marine bacteria from the liquid culturing medium and collect the cell mass to obtain the composition.

3. The method of claim 2, which further comprises the following steps:
(d) pulverizing the cell mass of the marine bacteria;
(e) digest the cell debris and dissolving the pigments by using a solvent; and
(f) removing the solvent to obtain zeaxanthin.

4. The method of claim 3, wherein the solvent is acetone, methanol, ethanol, ethyl acetate, isopropylalcohol, or cyclohexane.

5. The method of claim 3 or 4, wherein the zeaxanthin obtained from step (f) has at least 90%, preferably 95%, most preferred 97% (HPLC area %) of purity.

## Patentansprüche

1. Isolierter Bakterienstamm *Olleya marilimosa* mit der Hinterlegungsnummer CCTCC M2010201 zur Produktion von Zeaxanthin.

2. Verfahren zur Herstellung einer Zusammensetzung, die mikrobielles Zeaxanthin aus Meeresbakterien *Olleya marilimosa* mit der Hinterlegungsnummer CCTCC M2010201 umfasst, wobei es Folgendes umfasst:
(a) Anzüchten von Meeresbakterien *Olleya marilimosa* mit der Hinterlegungsnummer CCTCC M2010201 in einem flüssigen Nährmedium, um Pigmente zu bilden, die Zeaxanthin umfassen;
(b) Abtrennen der pigmenthaltigen Meeresbakterien-Zellmasse von dem flüssigen Nährmedium, und Auffangen der Zellmasse, um die Zusammensetzung zu erhalten.

3. Verfahren nach Anspruch 2, wobei es weiterhin die folgenden Schritte umfasst:
(d) Zerkleinern der Meeresbakterien-Zellmasse;
(e) Verdau der Zellbruchstücke und Inlösungbringen der Pigmente unter Verwendung eines Lösungsmittels; und
(f) Entfernen des Lösungsmittels, um Zeaxanthin zu erhalten.

4. Verfahren nach Anspruch 3, wobei es sich bei dem Lösungsmittel um Aceton, Methanol, Ethanol, Ethylacetat, Isopropylalkohol oder Cyclohexan handelt.

5. Verfahren nach Anspruch 3 oder 4, wobei das Zeaxanthin, welches in Schritt (f) erhalten wird, eine Reinheit von mindestens 90%, vorzugsweise 95%, mit dem größten Vorzug 97% (HPLC Flächen-%) aufweist.

## Revendications

1. Souche de bactérie isolée *Olleya marilimosa,* ayant le Numéro d'Accès de Dépôt CCTCC M2010201, pour la production de zéaxanthine.

2. Méthode de préparation d'une composition comprenant de la zéaxanthine microbienne issue de bactéries marines *Olleya marilimosa,* ayant le Numéro d'Accès de Dépôt CCTCC M2010201, comprenant :
(a) la culture de bactéries marines *Olleya marilimosa,* ayant le Numéro d'Accès de Dépôt CCTCC M2010201, dans un milieu de culture liquide afin de former des pigments comprenant de la zéaxanthine ;
(b) la séparation de la masse cellulaire contenant les pigments des bactéries marines à partir du milieu de culture liquide et la récupération de la masse cellulaire afin d'obtenir la composition.

3. Méthode selon la revendication 2, comprenant en outre les étapes suivantes :
(d) la pulvérisation de la masse cellulaire des bactéries marines ;
(e) la digestion des débris cellulaires et la solubilisation des pigments par l'utilisation d'un solvant ; et
(f) l'élimination du solvant afin d'obtenir la zéaxanthine.

4. Méthode selon la revendication 3, dans laquelle le solvant est l'acétone, le méthanol, l'éthanol, l'acétate d'éthyle, l'alcool isopropylique ou le cyclohexane.

5. Méthode selon la revendication 3 ou 4, dans laquelle la zéaxanthine obtenue à partir de l'étape (f) possède une pureté d'au moins 90%, préférablement de 95%, tout préférablement de 97% (% surface en HPLC).
